# EUROPEAN PATENT SPECIFICATION

(11) **EP 0 774 969 B1**
(45) Date of publication and mention of the grant of the patent: **19.02.2003**
(21) Application number: 95930118.5
(22) Date of filing: 07.08.1995
(51) Int. Cl.: A61K 31/495, A61K 31/435, A61K 31/425

(54) **HIV PROTEASE INHIBITOR COMBINATION**
KOMBINATIONEN VON HIV-PROTEASE INHIBITOREN
COMBINAISON DE L'INHIBITEUR DE LA PROTEASE DU VIH

(30) Priority: 11.08.1994 US 289474; 14.11.1994 US 339369; 20.07.1995 US 492461
(43) Date of publication of application: 28.05.1997
(73) Proprietor: Merck & Co., Inc., Rahway New Jersey 07065-0900 (US)
(72) Inventor: DEUTSCH, Paul, J., Rahway, NJ 07065 (US); EMINI, Emilio, A., Rahway, NJ 07065 (US); VACCA, Joseph, P., Rahway, NJ 07065 (US)
(74) Representative: Cole, William Gwyn
(86) International application number: US9509956
(87) International publication number: WO96004913

(56) References cited:
- EP-A- 0 346 847
- EP-A- 0 486 948
- EP-A- 0 541 168
- WO-A-92/08698
- WO-A-92/08700
- WO-A-94/14436
- PROC NATL ACAD SCI, 26 April 1994 USA, pages 4096-4100, VACCA JP; DORSEY BD; SCHLEIF WA; LEVIN RB; MCDANIEL SL; DARKE PL; ZUGAY J 'L-735,524: an orally bioavailable human immunodeficiency virus type 1'

## Description

This invention relates to a combination of two HIV protease inhibitors.

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is the extensive post-translational processing of precursor polyproteins by a virally encoded protease to generate mature viral proteins required for virus assembly and function. Inhibition of this processing prevents the production of normally infectious virus. For example, Kohl, N.E. et al., Proc. Nat'l Acad. Sci., 85, 4686 (1988) demonstrated that genetic inactivation of the HIV encoded protease resulted in the production of immature, non-infectious virus particles. These results indicate that inhibition of the HIV protease represents a viable method for the treatment of AIDS and the prevention or treatment of infection by HIV.

Nucleotide sequencing of HIV shows the presence of a pol gene in one open reading frame [Ratner, L. et al., Nature, 313, 277 (1985)]. Amino acid sequence homology provides evidence that the pol sequence encodes reverse transcriptase, an endonuclease and an HIV protease [Toh, H. et al., EMBO J. 4, 1267 (1985); Power, M.D. et al., Science, 231, 1567 (1986); Pearl, L.H. et al., Nature, 329, 351 (1987)].

The Compound A, of the structure: 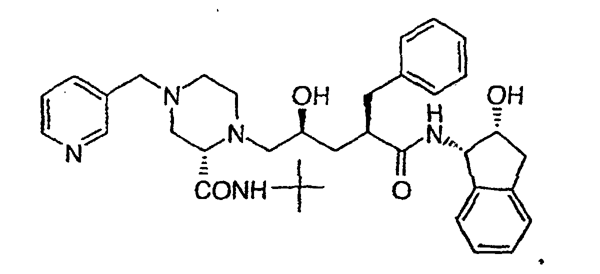 named N-(2(R)-hydroxy-1(S)-indanyl)-2(R)-phenylmethyl-4(S)-hydroxy-5-(1-(4-(3-pyridylmethyl)-2(S)-N'-(t-butylcarbamoyl)-piperazinyl))-pentaneamide, or pharmaceutically acceptable salt thereof, is a potent inhibitor of HIV protease and is useful in the treatment of AIDS or ARC, without substantial side effects or toxicity.

Applicants have discovered that administration of Compound J in combination with Compound B is useful for the treatment of AIDS or ARC.

Applicants demonstrate that the combination of compounds of this invention is an effective inhibitor of HIV protease.

In the present invention, applicants administer either simultaneously or alternatively the potent HIV protease inhibitor Compound A with an other potent HIV protease inhibitor, Compound B.

The combination in this invention is useful in the inhibition of HIV protease, the prevention of infection by HIV, the treatment of infection by HIV and in the treatment of AIDS and/or ARC, either as compounds, pharmaceutically acceptable salts (when appropriate), pharmaceutical composition ingredients, whether or not in combination with other antivirals, anti-infectives, immunomodulators, antibiotics or vaccines. Methods of treating AIDS, methods of preventing infection by HIV, and methods of treating infection by HIV are also disclosed.

This invention is concerned with the combination of certain compounds, or pharmaceutically acceptable salts thereof, in the inhibition of HIV protease, the prevention or treatment of infection by HIV and in the treatment of the resulting acquired immune deficiency syndrome (AIDS). The combination is defined as follows:

A combination comprising Compound A and Compound B.

The HIV protease inhibitor Compound A is synthesized by the protocol of EP 0541168, published 12 May 1993. The Compound L-735,524 is N-(2(R)-hydroxy-1(S)-indanyl)-2(R)-phenylmethyl-4-(S)-hydroxy-5-(1-(4-(3-pyridylmethyl)-2(S)-N'-(t-butylcarboxamido)-piperazinyl))-pentaneamide, or pharmaceutically acceptable salt thereof.

Compound B is: 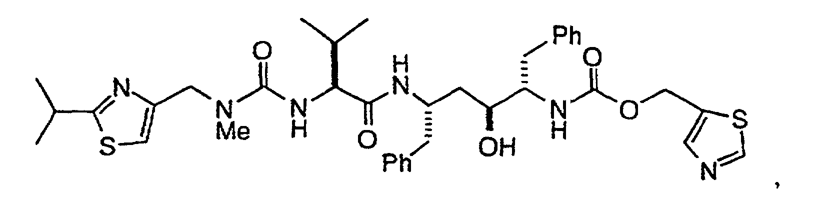 or pharmaceutically acceptable salt thereof. It is synthesized by the methods of EP 0486948, and PCT WO 94/14436.

The pharmaceutically-acceptable salts of the present invention (in the form of water- or oil-soluble or dispersible products) include the conventional non-toxic salts or the quaternary ammonium salts which are formed, e.g., from inorganic or organic acids. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxy-ethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Other pharmaceutically acceptable salts include the sulfate salt ethanolate and sulfate salts.

C₁₋₄ alkyl esters as prodrugs are included wherever appropriate.

The combination of compounds of the present invention is useful in the inhibition of HIV protease, the prevention or treatment of infection by human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymptomatic, and actual or potential exposure to HIV. For example, the compounds of this invention are useful in treating infection by HIV after suspected past exposure to HIV by, e.g., blood transfusion, exchange of body fluids, bites, accidental needle stick, or exposure to patient blood during surgery.

The combinations in this invention are also useful in the preparation and execution of screening assays for antiviral compounds. For example, the combinations in this invention are useful for isolating enzyme mutants, which are excellent screening tools for more powerful antiviral compounds. Furthermore, the combinations in this invention are useful in establishing or determining the binding site of other antivirals to HIV protease, e.g., by competitive inhibition. Thus the combinations in this invention are commercial products to be sold for these purposes.

For these purposes, the combinations of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically-effective amount of each compound in the combination of the present invention.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

The compounds of this invention can be administered to humans in the dosage ranges specific for each compound. Compound A or pharmaceutically acceptable salt thereof is administered orally in a dosage range between 40 mg and 4000 mg per day, divided into between one and four doses per day. A preferred oral dosage range for Compound A or pharmaceutically acceptable salt thereof is between 200 mg and 1000 mg administered three times per day. Compound B or pharmaceutically acceptable salt thereof is administered orally at a dosage range of between 100 mg and 4000 mg per day. A preferred oral dosage range for Compound B or pharmaceutically acceptable salt thereof is between 200 mg and 1000 mg administered three times per day. It will be of understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

### EXAMPLE 1

### Protocol for Pharmacokinetic Evaluation of Combination Therapy with Only Compound B

This is a multiple-dose, randomized, three-period, crossover-protocol in HIV-infected patients to evaluate the pharmacokinetics and safety of co-administration of Compound A and Compound B.

Twelve HIV-positive patients receive, in randomized order, three different treatments consisting of seven full days of dosing and one additional dose of: active Compound A with Compound B placebo (Treatment A); Compound A placebo with active Compound B (Treatment B); and active Compound A with active Compound B (Treatment C). The treatments are outlined in the following table:

| TREATMENT | BOTTLE NUMBER | DRUG | DOSE |
|---|---|---|---|
| A | 1 | Compound A | 600 mg q8h x 22 doses |
| | | | |
| | 2 | Compound B placebo | 3 capsules q8h x 22 doses |
| B | 1 | Compound A placebo | 3 capsules q8h x 22 doses |
| | | | |
| | 2 | Compound B | 600 mg q8h x 22 doses |
| C | 1 | Compound A | 600 mg q8h x 22 doses |
| | | | |
| | 2 | Compound B | 600 mg q8h x 22 doses |

Compound A is administered as three 200 mg capsules and Compound B as three 200 mg capsules. Placebo capsules match both the active Compound A and the active Compound B.

Compound A or matched placebo is consumed every eight hours. Potential subjects are evaluated for eligibility with a complete history, physical examination (including vital signs with orthostatic signs), 12-lead ECG, and laboratory screening (including a CD4 count) within approximately one month of the study start.

For analysis of safety during each treatment period, blood and urine for laboratory safety tests are obtained and physical examinations performed prior to the first dose (Day 1) and on the last day of dosing (Day 8) of each treatment period. Additionally, 12-lead ECGs are obtained prior to and one hour following the first dose as well as 1 hour following the last dose of each treatment period. Vital signs, including orthostatic signs are measured at frequently scheduled times on the first and last day of dosing. Subjects return on one interim day during the week of dosing (the same day of dosing for each treatment for an individual subject, either Day 3, 4, or 5) for observed dosing, observation for adverse effects, and monitoring of vital signs. Subjects maintain a Diary Card to record the times of ingestion of all doses and note any adverse experiences during each treatment period. A postprotocol evaluation for safety consisting of laboratory safety tests, physical examination, and ECG is performed 24 hours following the final treatment.

During Treatments A, B and C, blood is drawn for determination of plasma concentrations of Compound A and Compound B at 0, 0.25, 0.5, 0.75, 1, 1.5, 2, 3, 4, 6 and 8 hours following last dose (Day 8). Pharmacokinetic parameters to be analyzed include the maximum plasma concentration and the AUC (area under the concentration-time curve) for each drug.

### EXAMPLE 2

### Protocol for Combination Therapy with Only Compound B

In this protocol to show the antiviral activity of one regimen of Compound A given with Compound B in HIV-seronegative subjects, Compound A is administered at a dose of 600 mg three times a day and Compound B is administered at 600 mg three times a day. Antiviral activity is measured before and during combination therapy by measuring serum levels of the HIV p24 antigen, serum levels of HIV RNA, and CD4 lymphocyte counts.

## Claims

1. A combination of N-(2(R)-hydroxy-1(S)-indanyl)-2(R)-phenylmethyl-4(S)-hydroxy-5-(1-(4-(3-pyridylmethyl)-2(S)-N'-(t-butylcarbamoyl)piperazinyl))pentaneamide (Compound A), or a pharmaceutically acceptable salt thereof, and 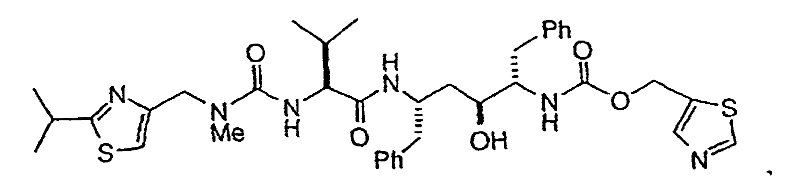 (Compound B), or a pharmaceutically acceptable salt thereof.

2. A combination as defined in claim 1 which employs capsules of Compound A and capsules of Compound B.

3. A combination according to claim 1 or 2 wherein Compound A, or the pharmaceutically acceptable salt thereof, is provided as an oral dose at a dosage range of between 40mg and 4000mg per day, divided between one and four doses per day.

4. A combination according to claim 3 wherein the dosage is between 200mg and 1000mg of Compound A, or a pharmaceutically acceptable salt thereof is provided.

5. A combination according to claim 1, 2, 3 or 4 wherein Compound B is provided as an oral dose at a dosage range of between 100mg and 4000mg per day.

6. A combination according to claim 5 wherein the dosage of Compound B is 100mg.

7. Use of a combination as defined in any of claims 1 to 6 for the manufacture of a medicament for inhibiting HIV protease, preventing infection by HIV, or for treating infection by HIV or for treating AIDS or ARC.

## Patentansprüche

1. Eine Kombination aus N-(2(R)-Hydroxy-1(S)-indanyl)-2(R)-phenylmethyl-4(S)-hydroxy-5-(1-(4-(3-pyridylmethyl)-2(S)-N'-(t-butylcarbamoyl)-piperazinyl))pentanamid (Verbindung A) oder einem pharmazeutisch annehmbaren Salz davon und 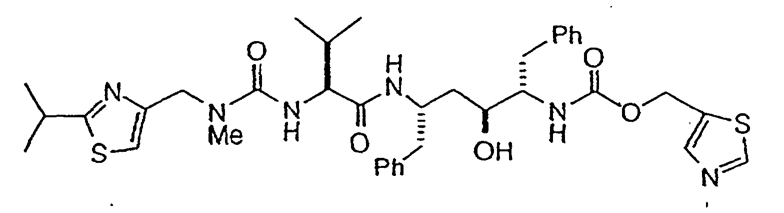 (Verbindung B) oder einem pharmazeutisch annehmbaren Salz davon.

2. Eine wie in Anspruch 1 definierte Kombination, die Kapseln von Verbindung A und Kapseln von Verbindung B einsetzt.

3. Eine Kombination gemäß Anspruch 1 oder 2, wobei Verbindung A oder das pharmazeutisch annehmbare Salz davon als eine orale Dosis mit einem Dosierungsbereich zwischen 40 mg und 4000 mg pro Tag, aufgeteilt auf ein bis vier Dosen pro Tag, bereitgestellt wird.

4. Eine Kombination gemäß Anspruch 3, wobei die bereitgestellte Dosierung zwischen 200 mg und 1000 mg von Verbindung A oder einem pharmazeutisch annehmbaren Salz davon beträgt.

5. Eine Kombination gemäß Anspruch 1, 2, 3 oder 4, wobei Verbindung B als eine orale Dosis mit einem Dosierungsbereich zwischen 100 mg und 4000 mg pro Tag bereitgestellt wird.

6. Eine Kombination gemäß Anspruch 5, wobei die Dosierung von Verbindung B 100 mg beträgt.

7. Die Verwendung einer wie in irgendeinem der Ansprüche 1 bis 6 definierten Kombination zur Herstellung eines Medikaments zur Inhibierung von HIV-Protease, Prävention einer HIV-Infektion oder zur Behandlung einer HIV-Infektion oder zur Behandlung von AIDS oder ARC.

## Revendications

1. Combinaison de N- (2(R)-hydroxy-1(S)-indanyle)-2(R)-phénylméthyle-4(S)-hydroxy-5-(1- (4- (3-pyridylméthyle)-2(S)-N'-(t-butylcarbamoyle)-pipérazinyle))pentaneamide (Composé A), ou d'un sel pharmaceutiquement acceptable de celui-ci, et de 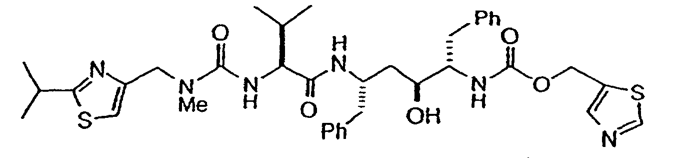 (Composé B), ou d'un sel pharmaceutiquement acceptable de celui-ci.

2. Combinaison selon la revendication 1, qui utilise des capsules de Composé A et des capsules de Composé B.

3. Combinaison selon la revendication 1 ou 2, dans laquelle le Composé A, ou le sel pharmaceutiquement acceptable de celui-ci, est fourni sous forme d'une dose orale dans une plage de dosage comprise entre 40 mg et 4000 mg par jour, divisée entre une et quatre doses par jour.

4. Combinaison selon la revendication 3, dans laquelle le dosage est compris entre 200 mg et 1000 mg du Composé A, ou d'un sel pharmaceutiquement acceptable de celui-ci.

5. Combinaison selon la revendication 1, 2, 3 ou 4, dans laquelle le Composé B est fourni sous forme d'une dose orale dans une plage de dosage comprise entre 100 mg et 4000 mg par jour.

6. Combinaison selon la revendication 5, dans laquelle le dosage du Composé B est de 100 mg.

7. Utilisation d'une combinaison selon l'une quelconque des revendications 1 à 6, pour la fabrication d'un médicament destiné à inhiber une protéase VIH, prévenir une infection par VIH, ou à traiter une infection par VIH ou à traiter le SIDA ou le para-SIDA (ARC).
